# EUROPEAN PATENT APPLICATION

(11) **EP 3 546 025 A2**
(43) Date of publication of application: **02.10.2019**
(21) Application number: 18213408.0
(22) Date of filing: 20.11.2012
(51) Int. Cl.: A61Q 17/04, A61K 8/26, A61K 8/37, A61K 8/04, A61K 8/31, A61K 8/34, A61K 8/891, A61K 8/92

(54) **USE OF OLEOGELS IN UV ABSORBER COMPOSITIONS**

(30) Priority: 23.11.2011 US 201161563048 P; 23.11.2011 EP 11190241
(62) Divisional of application: 12788208.2
(71) Applicant: BASF SE, 67056 Ludwigshafen am Rhein (DE)
(72) Inventor: HERZOG, Bernd, 79639 Grenzach-Wyhlen (DE); DESHAYES, Cyrille, 68128 Rosenau (FR)
(74) Representative: BASF IP Association

(57) **Abstract**

Disclosed is the use of oleogels (a) for increasing the sun protection factor of sunscreens comprising at least one organic or inorganic UV filter (b).

## Description

Most of nowadays skin-care products are based on emulsions (crèmes, lotions and milks). Emulsions are mixtures of oils or oil soluble substances and water or water soluble components and represent the most important product type of skin care products.

In addition there still exist skin-care products with only an oily phase which are preferably recommended for problem skins and are therefore used in the dermatological cosmetics. In terms of applicability an oily skin surface is inconvenient and leaves an unpleasant feeling. These problems have been solved with the development of emulsions which increase the penetration of lipids into the skin and reduce the concentration of oils and lipids in the products. However, the presence of water in the formulations involved new problems: it became necessary to preserve the aqueous phase of the emulsions, some ingredients are sensitive to water and it was indispensible to stabilize the emulsions against long-term influences of temperature and storage.

These problems were successfully resolved with the use of indifferent ingredients like mineral oils and waxes and additional additives. Many of these additives are not tolerated by sensitive skin. Individuals with neurodermitic skin e.g. will not tolerate the long-term use of ethoxylated alcohols which belong to the most widely used emulsifiers. Allergy sufferers have problems with appropriate preservatives etc..

Since particularly individuals with skin barrier disorders are dependent on physiological lipids in high dosage problem solutions for this specific group gain more and more importance. These lipids still are based on the application of appropriate oils, a minimum content of additives and convenient application features. For this purpose oleogels are recommended which are also known as lipogels.

Oleogels are semisolid, semitransparent or transparent systems containing so called gel formers and an oil or a lipid as non-continuous (stationary) phase. The gel former develops a three-dimensional meshwork wherein the oil is immobilized. This typical structure can be compared with a liquid-impregnated sponge wherein the sponge represents the gel former thus enabling to assimilate large amounts of lipids. In contrast to hydrogels oleogels are generally free of water.

Sun screen compositions comprising an oil phase have a significant influence on the properties of the UV filters, i.e. changes in both the wave length of maximum absorbance (λₘₐₓ) and molar absorptivity (ε) can be observed for many of the sunscreen systems.

Oleogels can therefore advantageously be used in sunscreens. Surprisingly it was found that the use of oleogels in sunscreens which contain at least one UV filter will increase the sun protection factor of sunscreen compositions.

Therefore, the present invention relates to the use of oleogels (a) for increasing the sun protection factor of sunscreens comprising at least one organic or inorganic UV filter (b).

The stationary phase (oil or lipid) of the oleogels (a) are preferably selected from
(sp₁) Guerbet alcohols,
(sp₂) esters of linear C₆-C₂₄ fatty acids with linear C₃-C₂₄ alcohols,
(sp₃) esters of branched C₆-C₁₃carboxylic acids with linear C₆-C₂₄ fatty alcohols,
(sp₄) esters of linear C₆-C₂₄ fatty acids with branched alcohols,
(sp₅) esters of hydroxycarboxylic acids with linear or branched C₆-C₂₂ fatty alcohols,
(sp₆) esters of linear and/or branched fatty acids with polyhydric alcohols,
(sp₇) triglycerides based on C₆-C₁₀ fatty acids,
(sps) liquid mono-/di-/tri-glyceride mixtures based on C₆-C₁₈ fatty acids,
(sp₉) esters of C₆-C₂₄ fatty alcohols and/or Guerbet alcohols with aromatic carboxylic acids,
(sp₁₀) esters of C₂-C₁₂dicarboxylic acids with linear or branched alcohols having from 1 to 22 carbon atoms or polyols having from 2 to 10 carbon atoms and from 2 to 6 hydroxy groups,
(sp₁₁) vegetable oils,
(sp₁₂) branched primary alcohols,
(sp₁₃) substituted cyclohexanes,
(sp₁₄) linear and branched C₆-C₂₂ fatty alcohol carbonates,
(sp₁₅) Guerbet carbonates,
(sp₁₆) esters of benzoic acid with linear and/or branched C₆-C₂₂alcohols,
(sp₁₇) linear or branched, symmetric or asymmetric dialkyl ethers having a total of from 12 to 36 carbon atoms,
(sp₁₈) silicone oils,
(sp₁₉) aliphatic or naphthenic hydrocarbons,
(sp₂₀) monoesters of fatty acids with alcohols having from 3 to 24 carbon atoms,
(sp₂₁) isopropyl myristate,
(sp₂₂) isononanoic acid C₁₆-C₁₈alkyl esters,
(sp₂₃) stearic acid 2-ethylhexyl ester,
(sp₂₄) cetyl oleate,
(sp₂₅) glycerol tricaprylate,
(sp₂₆) coconut fatty alcohol caprinate/caprylate
(sp₂₇) n-butyl stearate
(sp₂₈) dicarboxylic acid esters,
(sp₂₉) diol esters,
(sp₃₀) polyols and
(sp₃₁) di- and/or trivalent metal salts.

Most preferably the stationary phase of the oleogel (a) is selected from Dibutyl Adipate Diethylhexyl Carbonate.

The gel former of the oleogels (a) according to the present invention is preferably selected from
(gf₁) stearalkonium hectorite (bentonite),
(gf₂) gelatine,
(gf₃) silica,
(gf₄) montmorillonite,
(gf₅) monoglyceridees and diglycerides,
(gf₆) polysaccharides,
(gf₇) pectins and
(gf₈) specific polymers.

Most preferably the gel former of the oleogels (a) is selected from stearalkonium hectorrite in combination with propylene carbonate.

The UV filters (b) according to the present invention are preferably selected from
(b₁) triazine derivatives,
(b₂) hydroxybenzophenone derivatives,
(b₃) Methoxydibenzoylmethane derivatives,
(b₄) substituted acrylates,
(b₅) cinnamic acid derivatives,
(b₆) salicylic acid derivatives,
(b₇) benzotriazole derivatives; and
(b₈) inorganic pigments.

More preferably the triazine derivatives (b₁) are selected from compounds of formula 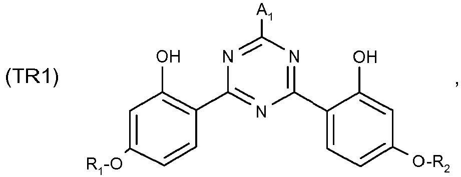 wherein
R₁ and R₂ are each independently of the other C₁-C₁₈alkyl; C₂-C₁₈alkenyl; or a radical of formula -CH₂-CH(-OH)-CH₂-O-T₁;
A₁ is a radical of formula 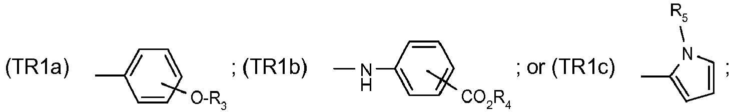
   - R₃: is hydrogen; or C₁-C₁₀alkyl,
   - R₄: is hydrogen; M; or C₁-C₅alkyl;
   - R₅: is C₁-C₁₈alkyl; and
   - M: is a metal cation.

Most preferred representative of the triazine derivatives (b₁) is Bis-ethylhexyloxy methoxyphenyl triazine (BEMT) corresponding to formula

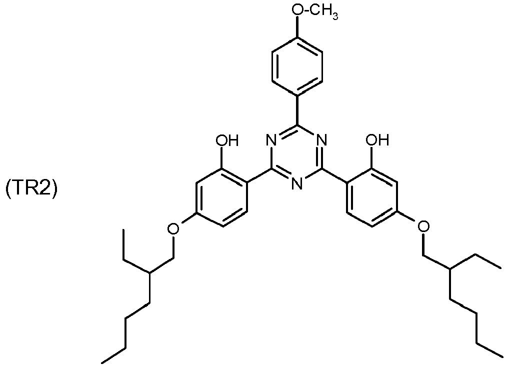

Also preferred are triazine derivatives (b₁) which correspond to the compounds of formula 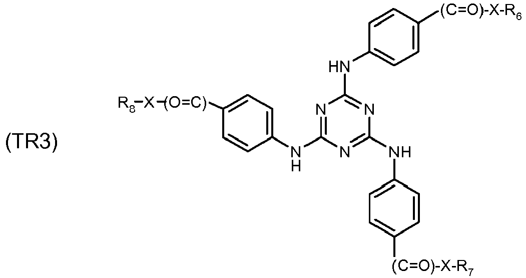 wherein
- R₆, R₇ and R₈: independently of each other are C₁-C₂₀alkyl, C₆-C₁₀aryl, heteroaryl, optionally
- su: bstituted;
- X: is O; or NR₉; and
- R₉: is hydrogen; C₁-C₂₀alkyl, C₆-C₁₀aryl, heteroaryl, optionally substituted,

Most preferred representative is Ethylhexyl triazone (EHT) corresponding to formula 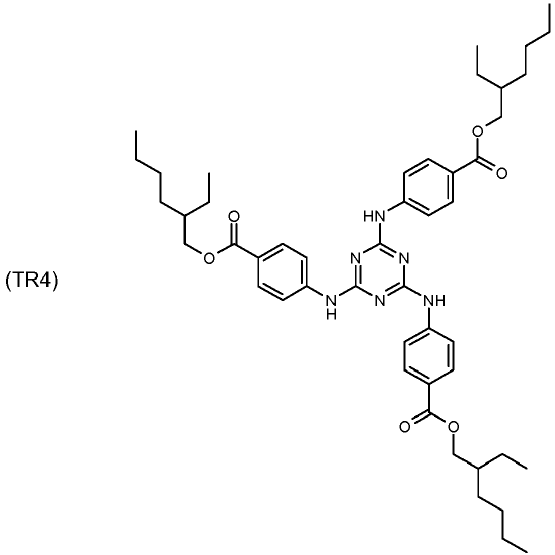 or Diethylhexyl butamido triazone (DBT) corresponding to formula

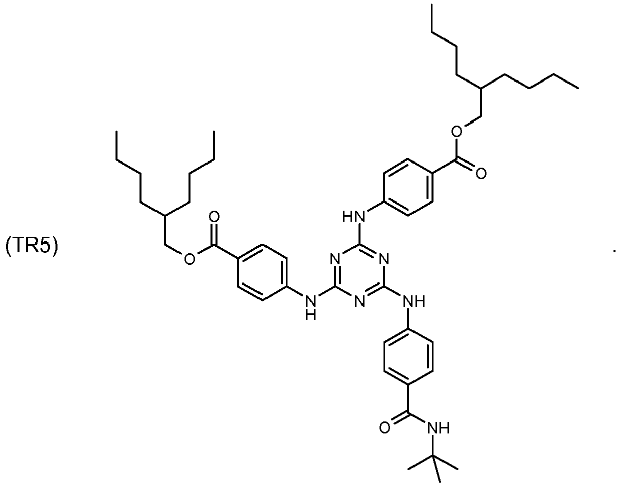

Preferably the hydroxybenzophenone derivatives (b₂) are amino-substituted hydroxybenzophenones corresponding to the formula 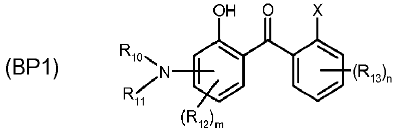 wherein
R₁₀ and R₁₁, independently from each other are hydrogen, C₁-C₂₀alkyl, C₂-C₁₀alkenyl, C₃-C₁₀cycloalkyl, C₃-C₁₀cycloalkenyl, where the substituents R₁₀ and R₁₁ together with the nitrogen atom to which they are bonded may form a 5- or 6-membered ring;
R₁₂ and R₁₃ independently from each other are C₁C₂₀alkyl; C₂-C₁₀alkenyl; C₃-C₁₀cycloalkyl; C₃-C₁₀cycloalkenyl; C₁-C₁₂alkoxy; C₁-C₂₀alkoxycarbonyl; C₁-C₁₂alkylamino; C₁-C₁₂dialkylamino; aryl; heteroaryl, optionally substituted; substituents which confer solubility in water, chosen from the group consisting of a nitrile group, carboxylate, sulfonate and ammonium radicals;
X is hydrogen; COOR₁₄; or CONR₁₅R₁₆;
R₁₄ to R₁₆ are hydrogen; C₁-C₂₀alkyl; C₂-C₁₀alkenyl; C₃-C₁₀cycloalkyl; C₃-C₁₀cycloalkenyl, or -(Y-O)o-Z, C₆-C₁₀aryl;
Y is -(CH₂)₂-; -(CH₂)₃-, -(CH₂)₄-; or -CH(CH₃)-CH₂-;
Z is -CH₂-CH₃; -CH₂-CH₂-CH₃; -CH₂-CH₂-CH₂-CH₃; or -CH(CH₃)-CH₃;
m is from 0 to 3;
n is from 0 to 4; and
o is from 1 to 20.

More preferred representative of amino-substituted hydroxybenzophenones (b₂) is Diethylamino hydroxybenzoyl hexyl benzoate (DHHB) corresponding to formula

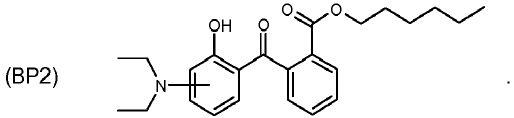

Further preferred representative of (b₂) is Benzophenone-3 (B-3) corresponding to formula

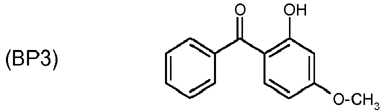

Preferred representative of the methoxydibenzoylmethane derivatives (b₃) is Butyl methoxy dibenzoyl methane (BMDBM) corresponding the formula

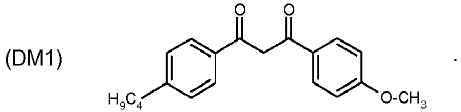

Preferred representative of substituted acrylates (b₄) is Octocrylene (OCR) corresponding to the formula

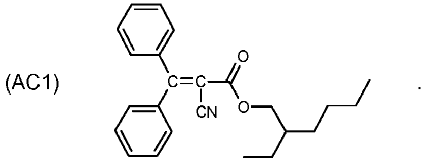

Preferred representative of cinnamic acid derivatives (b₅) is Ethylhexylmethoxy cinnamate (EHMC) corresponding to the formula

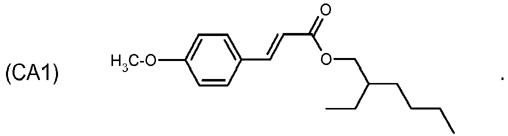

Preferred representative of salicylic acid derivatives (b₆) is Ethylhexyl salicylate corresponding to the formula

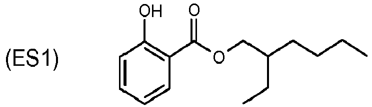

Preferred representative of benzotriazole derivatives (b₇) is Drometrizole Trisiloxane corresponding to formula

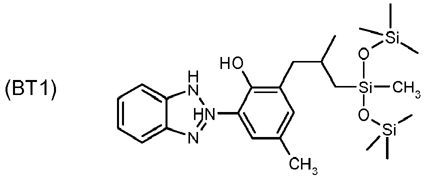

Preferably the inorganic pigments (b₈) are selected from oil-dispersed TiO₂.

More preferably the UV filters (b₁) - (b₈) are used in mixtures.

The oil-soluble or oil-miscible UV filters commercially used worldwide are listed in Table 1 below. All of them might be used in any combination within oleogel formulations. The concentrations may be varied as well according to the registration status in the different regions.

| Table 1: Oil-soluble or oil-miscible UV filters | | | | | | |
|---|---|---|---|---|---|---|
| INCI* name and abbreviation | | | Registration status, max. incorporation level | | | |
| | USAN* | λₘₐₓ | USA | Japan | Europe | Australia |
| Ethylhexyl dimethyl PABA (ED-PABA) | Padimate-O | 311 nm | 8% | 10% | 8% | 8% |
| Homomenthyl salicylate (HMS) | Homosalate | 306 nm | 15% | 10% | 10% | 15% |
| Ethylhexyl salicylate (EHS) | Octisalate | 305 nm | 5% | 10% | 5% | 5% |
| Isoamylmethoxy cinnamate (IMC) | Amiloxate | 308 nm | TEA* | 10% | 10% | 10% |
| Ethylhexylmethoxy cinnamate (EHMC) | Octinoxate | 311 nm | 7.5% | 20% | 10% | 10% |
| Octocrylene (OCR) | Octocrylene | 303 nm | 10% | 10% | 10% | 10% |
| Polysilicone 15 (BMP) | - | 312 nm | - | 10% | 10% | 10% |
| Benzophenone-3 (B-3) | Oxybenzone | 324 nm | 6% | 5% | 10% | 10% |
| 4-Methyl benzylidene camphor (MBC) | Enzacamene | 300 nm | TEA* | - | 4% | 4% |
| Ethylhexyl triazone (EHT) | - | 314 nm | TEA* | 3% | 5% | 5% |
| Diethylhexyl butamido triazone (DBT) | - | 311 nm | - | - | 10% | - |
| Menthyl anthranilate (MA) | Meradimate | 336 nm | 5% | - | - | 5% |
| Butyl methoxy dibenzoyl methane (BMDBM) | Avobenzone | 357 nm | 3% | 10% | 5% | 5% |
| Diethylamino hydroxybenzoyl hexyl benzoate (DHHB) | - | 354 nm | - | - | 10% | - |
| Drometrizole Trisiloxane (DTS) | - | 303 & 341 nm | - | 10% | 15% | 15% |
| Bis-ethylhexyloxy methoxyphenyl triazine (BEMT) | Bemotrizinol | 310 & 343 nm | TEA* | 3% | 10% | 10% |

Examples of mixtures of 2 UV-filters which are preferably used according to the present invention:

| | |
|---|---|
| (Mix01) | compound (TR2) and compound (CA1); |
| (Mix02) | compound (TR2) and compound (TR4); |
| (Mix03) | compound (TR2) and compound (TR5); |
| (Mix04) | compound (TR2) and compound (AC1); |
| (Mix05) | compound (BP1) and compound (CA1); |
| (Mix06) | compound (BP1) and compound (TR4); |
| (Mix07) | compound (BP1) and compound (TR5); |
| (Mix08) | compound (BP1) and compound (AC1); |
| (Mix09) | compound (TR2) and compound (BP1); |
| (Mix10) | compound (TR4) and compound (CA1); |
| (Mix11) | compound (TR5) and compound (CA1); |
| (Mix12) | compound (DM1) and compound (TR4); |
| (Mix13) | compound (DM1) and compound (TR5); |
| (Mix14) | compound (DM1) and compound (AC1); |
| (Mix15) | oil-dispersed TiO₂ and compound (TR2): |
| (Mix16) | oil-dispersed TiO₂ and compound (BP1); |
| (Mix17) | oil-dispersed TiO₂ and compound (TR4); |
| (Mix18) | oil-dispersed TiO₂ and compound (TR5); |
| (Mix19) | oil-dispersed TiO₂ and compound (CA1); |
| (Mix20) | oil-dispersed TiO₂ and compound (AC1); |
| (Mix21) | oil-dispersed ZnO and compound (TR2); |
| (Mix22) | oil-dispersed ZnO and compound (TR2); |
| (Mix23) | oil-dispersed ZnO and compound (BP1); |
| (Mix24) | oil-dispersed ZnO and compound (TR4); |
| (Mix25) | oil-dispersed ZnO and compound (TR5); |
| (Mix26) | oil-dispersed TiO₂ and oil-dispersed ZnO; |
| (Mix27) | compound (DM1) and compound (AC1); |
| (Mix28) | compound (DM1) and compound (TR2); |

Examples of mixtures of 3 UV-filters which are preferably used according to the present invention:

| | |
|---|---|
| (Mix29): | compound (DM1) and compound (AC1); and compound (TR2); |
| (Mix30): | compound (DM1) and compound (AC1); and compound (TR4); |
| (Mix31): | compound (DM1) and compound (AC1); and compound (TR5); |
| (Mix32): | compound (CA1) and compound (BP2); and compound (TR4); |
| (Mix33): | compound (CA1) and compound (BP2); and compound (TR5); |
| (Mix34): | compound (CA1) and compound (BP2); and compound (TR2); |
| (Mix35): | compound (DM1) and compound (BP3); and compound (AC1); |
| (Mix36): | compound (CA1) and compound (BP2); and compound (BP3); |
| (Mix37): | compound (TR2) and compound (TR4); and compound (BP2); |

Examples of mixtures of 4 UV-filters which are preferably used according to the present invention:

| | |
|---|---|
| (Mix38): | compound (DM1) and compound (AC1); and compound (TR2); and compound (TR4); |
| (Mix39): | compound (DM1) and compound (AC1); and compound (TR2); and compound (TR5); |
| (Mix40): | compound (CA1) and compound (BP2); and compound (TR2); and compound (TR4); |
| (Mix41): | compound (CA1) and compound (BP2); and compound (TR2); and compound (TR5); |
| (Mix42): | compound (CA1) and compound (BP2); and compound (BP3); and compound (TR2); |
| (Mix43): | compound (CA1) and compound (BP2); and compound (TR2); and compound (TR5). |

Examples of mixtures of 5 UV-filters which are preferably used according to the present invention:

| | |
|---|---|
| (Mix44): | compound (CA1) and compound (BP2); and compound (TR2); and compound (TR4); and compound (ES1); |
| (Mix45): | compound (CA1) and compound (BP2); and compound (TR2); and compound (TR5); and compound (ES1); |
| (Mix46): | compound (DM1) and compound (AC1); and compound (TR2); and compound (TR4); and compound (ES1); |
| (Mix47): | compound (CA1) and compound (AC1); and compound (TR2); and compound (TR5); and compound (ES1). |

Most preferably mixtures of (Mix37) comprising the compound (TR2) and compound (TR4) and compound (BP2) are used.

Most preferably the present invention relates to use of a combination of
(a) the oleogel formed from (gf₁) stearalkonium hectorite and the stationary phase selected from Dibutyl Adipate and Diethylhexyl Carbonate; and
(b) (Mix37) comprising compound (TR2) and compound (TR4) and compound (BP2).

Oils as representatives for the non-continuous phase are for example (sp₁) Guerbet alcohols, based on fatty alcohols having from 6 to 18, preferably from 8 to 10, carbon atoms; (sp₂) esters of linear C₆-C₂₄ fatty acids with linear C₃-C₂₄ alcohols, (sp₃) esters of branched C₆-C₁₃carboxylic acids with linear C₆-C₂₄ fatty alcohols, (sp₄) esters of linear C₆-C₂₄ fatty acids with branched alcohols, especially 2-ethylhexanol, (sp₅) esters of hydroxycarboxylic acids with linear or branched C₆-C₂₂ fatty alcohols, especially dioctyl malates; (sp₆) esters of linear and/or branched fatty acids with polyhydric alcohols, (for example propylene glycol, dimer diol or trimer triol) and/or Guerbet alcohols; (sp₇) triglycerides based on C₆-C₁₀ fatty acids; (sp₈) liquid mono-/di-/tri-glyceride mixtures based on C₆-C₁₈ fatty acids; (sp₉) esters of C₆-C₂₄ fatty alcohols and/or Guerbet alcohols with aromatic carboxylic acids, especially benzoic acid; (sp₁₀) esters of C₂-C₁₂dicarboxylic acids with linear or branched alcohols having from 1 to 22 carbon atoms or polyols having from 2 to 10 carbon atoms and from 2 to 6 hydroxy groups; (sp₁₁) vegetable oils, such as sunflower oil, olive oil, soybean oil, rapeseed oil, almond oil, jojoba oil, orange oil, wheat germ oil, peach kernel oil and the liquid components of coconut oil; (sp₁₂) branched primary alcohols; (sp₁₃) substituted cyclohexanes; (sp₁₄) linear and branched C₆-C₂₂ fatty alcohol carbonates; (sp₁₅) Guerbet carbonates; (sp₁₆) esters of benzoic acid with linear and/or branched C₆-C₂₂alcohols (e.g. Finsolv® TN); (sp₁₇) linear or branched, symmetric or asymmetric dialkyl ethers having a total of from 12 to 36 carbon atoms, especially from 12 to 24 carbon atoms, for example di-n-octyl ether, di-n-decyl ether, di-n-nonyl ether, di-n-undecyl ether, di-n-dodecyl ether, n-hexyl n-octyl ether, n-octyl n-decyl ether, n-decyl n-undecyl ether, n-undecyl n-dodecyl ether, n-hexyl n-undecyl ether, di-tert-butyl ether, diisopentyl ether, di-3-ethyldecyl ether, tert-butyl n-octyl ether, isopentyl n-octyl ether and 2-methyl pentyl-n-octyl ether; (sp₁₈) silicone oils; (sp₁₉) aliphatic or naphthenic hydrocarbons; (sp₂₀) monoesters of fatty acids with alcohols having from 3 to 24 carbon atoms. That group of substances comprises the esterification products of fatty acids having from 8 to 24 carbon atoms, for example caproic acid, caprylic acid, 2-ethylhexanoic acid, capric acid, lauric acid, isotridecanoic acid, myristic acid, palmitic acid, palmitoleic acid, stearic acid, isostearic acid, oleic acid, elaidic acid, petroselinic acid, linoleic acid, linolenic acid, elaeostearic acid, arachidic acid, gadoleic acid, behenic acid and erucic acid and technical-grade mixtures thereof (obtained, for example, in the pressure removal of natural fats and oils, in the reduction of aldehydes from Roelen's oxosynthesis or in the dimerisation of unsaturated fatty acids) with alcohols, for example isopropyl alcohol, caproic alcohol, capryl alcohol, 2-ethylhexyl alcohol, capric alcohol, lauryl alcohol, isotridecyl alcohol, myristyl alcohol, cetyl alcohol, palmoleyl alcohol, stearyl alcohol, isostearyl alcohol, oleyl alcohol, elaidyl alcohol, petroselinyl alcohol, linoyl alcohol, linolenyl alcohol, elaeostearyl alcohol, arachidyl alcohol, gadoleyl alcohol, behenyl alcohol, erucyl alcohol and brassidyl alcohol and technical-grade mixtures thereof (obtained, for example, in the high-pressure hydrogenation of technical-grade methyl esters based on fats and oils or aldehydes from Roelen's oxosynthesis and as monomer fractions in the dimerisation of unsaturated fatty alcohols); (sp₂₁) isopropyl myristate; (sp₂₂) isononanoic acid C₁₆-C₁₈alkyl esters, (sp₂₃) stearic acid 2-ethylhexyl ester; (sp₂₄) cetyl oleate; (sp₂₅) glycerol tricaprylate; (sp₂₆) coconut fatty alcohol caprinate/caprylate; (sp₂₇) n-butyl stearate; (sp₂₈) dicarboxylic acid esters, such as di-n-butyl adipate, di(2-ethylhexyl) adipate, di(2-ethylhexyl) succinate and diisotridecyl acetate; (sp₂₉) diol esters, such as ethylene glycol dioleate, ethylene glycol diisotridecanoate, propylene glycol di(2-ethylhexanoate), propylene glycol diisostearate, propylene glycol dipelargonate, butanediol diisostearate and neopentyl glycol dicaprylate; (sp₃₀) polyols like ethanol, isopropanol, propylene glycol, hexylene glycol, glycerol and sorbitol; (sp₃₁) di- and/or trivalent metal salts (alkaline earth metal, Al³⁺ *inter alia*) of one or more alkyl carboxylic acids.

Preferred oil components of the Oleogels as used in the present invention are Dibutyl Adipate and Diethylhexyl Carbonate.

The oil components can be used in an amount of, for example, from 1 to 60 % by weight, especially from 5 to 50 % by weight and preferably from 10 to 35 % by weight, based on the total weight of the composition.

The respective formation of the oleogel is carried out according to operating conditions known to those skilled in the art. Reference will be made in particular to the examples below.

In particular, depending on the composition envisaged, the UV absorber(s) (b) can be added to the oleogel before preparing the oleogel of substantially uniform appearance. It is also possible to add the UV absorber(s) (b) to the preformed stable oleogel according to the invention, with stirring, the UV absorber(s) (b) thus being distributed in the Oleogel.

Needless to say, a person skilled in the art will know to take into account the characteristics of the UV absorber(s) used, adapting the operating conditions of the process, in particular the temperature, so as possibly not to adversely affect the properties of the active ingredient(s).

Lastly, the subject of the present invention relates to a stable cosmetic composition, characterized in that it comprises
(a) a stable oleogel and
(b) at least one organic or inorganic UV filter.

The sunscreen composition according to the present invention is especially useful for the protection of organic materials that are sensitive to ultraviolet light, especially human and animal skin and hair, against the action of UV radiation. Such UV filter combinations are accordingly suitable as light-protective agents in cosmetic, pharmaceutical and veterinary medicine preparations.

The cosmetic preparation may also comprise, in addition to the UV absorber combinations according to the invention, one or more further UV protective agents of the following substance classes:
p-aminobenzoic acid derivatives, benzophenone derivatives, 3-imidazol-4-yl acrylic acid and esters; benzofuran derivatives, polymeric UV absorbers, cinnamic acid derivatives,
camphor derivatives, menthyl o-aminobenzoate; merocyanine derivatives; or encapsulated UV absorbers.

The UV absorbers described in "Sunscreens", Eds. N.J. Lowe, N.A.Shaath, Marcel Dekker, Inc., New York and Basle or in Cosmetics & Toiletries (107), 50ff (1992) also can be used as additional UV protective substances.

Special preference is given to the light-protective agents indicated in the following Table 2:

| Table 2: Suitable UV filter substances and adjuvants which can be additionally used with the UV absorbers of formula MBM-01 - MBM-12 according to the present invention | | |
|---|---|---|
| No. | Chemical Name | CAS No. |
| 1 | (+/-)-1,7,7-trimethyl-3-[(4-methylphenyl)methylene]bicyclo-[2.2.1]heptan-2-one; p-methyl benzylidene camphor | 36861-47-9 |
| 2 | 1,7,7-trimethyl-3-(phenylmethylene)bicyclo[2.2.1]heptan-2-one; benzylidene camphor | 15087-24-8 |
| 3 | (2-Hydroxy-4-methoxyphenyl)(4-methylphenyl)methanone | 1641-17-4 |
| 4 | 2,4-dihydroxybenzophenone | 131-56-6 |
| 5 | 2,2',4,4'-tetrahydroxybenzophenone | 131-55-5 |
| 6 | 2-Hydroxy-4-methoxy benzophenone; | 131-57-7 |
| 7 | 2-Hydroxy-4-methoxy benzophenone-5-sulfonic acid | 4065-45-6 |
| 8 | 2,2'-dihydroxy-4,4'-dimethoxybenzophenone | 131-54-4 |
| 9 | 2,2'-Dihydroxy-4-methoxybenzophenone | 131-53-3 |
| 10 | Alpha-(2-oxoborn-3-ylidene)toluene-4-sulphonic acid and its salts (Mexoryl SL) | 56039-58-8 |
| 12 | Methyl N,N,N-trimethyl-4-[(4,7,7-trimethyl-3-oxobicyclo[2,2,1]hept-2-ylidene)methyl]anilinium sulphate (Mexoryl SO) | 52793-97-2 |
| 14 | 2-ethylhexyl 4-(dimethylamino)benzoate | 21245-02-3 |
| 16 | 4-aminobenzoic acid | 150-13-0 |
| 17 | Benzoic acid, 4-amino-, ethyl ester, polymer with oxirane | 113010-52-9 |
| 18 | 2-Propenamide, N-[[4-[(4,7,7-trimethyl-3-oxobicyclo[2.2.1]hept-2-ylidene)methyl]phenyl]methyl]-, homopolymer | 147897-12-9 |
| 19 | Triethanolamine salicylate | 2174-16-5 |
| 20 | 3,3'-(1,4-phenylenedimethylene)bis[7,7-dimethyl-2-oxo-bicyclo[2.2.1]heptane-1methanesulfonic acid] (Cibafast H) | 90457-82-2 |
| 22 | Phenol, 2-(2H-benzotriazol-2-yl)-4-methyl-6-[2-methyl-3-[1,3,3,3-tetramethyl-1-[(trimethylsilyl)oxy]disiloxanyl]propyl]-; drometrizole trisiloxane (Mexoryl XL) | 155633-54-8 |
| 23 | Dimethicodiethylbenzalmalonate; Polysilicone 15 (Parsol SLX) | 207574-74-1 |
| 24 | Benzenesulfonic acid, 3-(2H-benzotriazol-2-yl)-4-hydroxy-5-(1-methylpropyl)-, monosodium salt (Tinogard HS) | 92484-48-5 |
| 25 | 1-Dodecanaminium, N-[3-[[4-(dimethylamino)benzoyl]amino]-propyl]N,N-dimethyl-, salt with 4-methylbenzenesulfonic acid (1:1) (Escalol HP610) | 156679-41-3 |
| 26 | 1-Propanaminium, N,N,N-trimethyl-3-[(1-oxo-3-phenyl-2-propenyl)-amino]-, chloride | 177190-98-6 |
| 27 | 1H-Benzimidazole-4,6-disulfonic acid, 2,2'-(1,4-phenylene)bis- | 170864-82-1 |
| 28 | 1-Propanaminium, 3-[[3-[3-(2H-benzotriazol-2-yl)-5-(1,1-dimethylethyl)-4-hydroxyphenyl]-1-oxopropyl]amino]-N,N-diethyl-N-methyl-, methyl sulfate (salt) | 340964-15-0 |
| 29 | 2-Propenoic acid, 3-(1H-imidazol-4-yl)- | 104-98-3 |
| 30 | Benzoic acid, 2-hydroxy-, [4-(1-methylethyl)phenyl]methyl ester | 94134-93-7 |
| 31 | 1,2,3-Propanetriol, 1-(4-aminobenzoate) (Glyceryl PABA) | 136-44-7 |
| 32 | Benzeneacetic acid, 3,4-dimethoxy-a-oxo- | 4732-70-1 |
| 33 | 2-Propenoic acid, 2-cyano-3,3-diphenyl-, ethyl ester | 5232-99-5 |
| 34 | Anthralinic acid, p-menth-3-yl ester | 134-09-8 |
| 35 | sterols (cholesterol, lanosterol, phytosterols), as described in WO0341675 | |
| 36 | mycosporines and/or mycosporine-like amino acids as described in WO2002039974, e.g. Helioguard 365 from Milbelle AG, isolated mycosporine like amino acids from the red alga porphyra umbilicalis (INCI: Porphyra Umbilicalis) that are encapsulated into liposomes) | |
| 37 | alpha-lipoic-acid as described in DE 10229995 | |
| 38 | synthetic organic polymers as described in EP 1 371 358, [0033]-[0041] | |
| 39 | phyllosilicates as described in EP 1371357 [0034]-[0037] | |
| 40 | silica compounds as described in EP1371356, [0033]-[0041] | |
| 42 | latex particles as described in DE10138496 [0027]-[0040] | |
| 43 | 1H-Benzimidazole-4,6-disulfonic acid, 2,2'-(1,4-phenylene)bis-, disodium salt; Bisimidazylate (Neo Heliopan APC) | 180898-37-7 |
| 44 | Di-2-ethylhexyl-3,5-dimethoxy-4-hydroxy-benzalmalonate (Oxynex ST, EMD Chemicals, as described in US 20040247536) | |
| 45 | T-LiteTM MAX: Titanium Dioxide (and) Dimethoxydiphenylsilane (and) Triethoxycaprylylsilane Crosspolymer (and) Hydrated Silica (and) Aluminum Hydroxyde | |
| 46 | T-Lite SF: Titanium Dioxide (and) Aluminum Hydroxide (and) Dimethicone/Methicone Copolymer | |
| 47 | T-Lite SF-S: Titanium Dioxide (and) Hydrated Silica (and) Dimethicone/ Methicone Copolymer (and) Aluminum Hydroxide | |
| 48 | Z-COTE® MAX: Zinc Oxide (and) Diphenyl Capryl Methicone | |
| 49 | Z-COTE HP1: Zinc Oxide (and) Triethoxycaprylylsilane | |
| 50 | 1,1-[(2,2'-Dimethylpropoxy)carbonyl]-4,4-diphenyl-1,3-butadiene | 363602-15-7 |
| 51 | UV filter capsules containing an organic sunscreen as described in DE102007035567 or WO 2009012871 | |

In addition, BEMT (Tinosorb S, Bis-Ethylhexyloxyphenol Methoxyphenyl Triazine) encapsulated in a polymer matrix, for example in PMMA, as described in IP.com Journal (2009), 9(1B), 17, can also be used as additional UV protective substance.

The following compounds can also be used as additional UV protective substances: Merocyanine derivatives as described in WO 2004/006878: 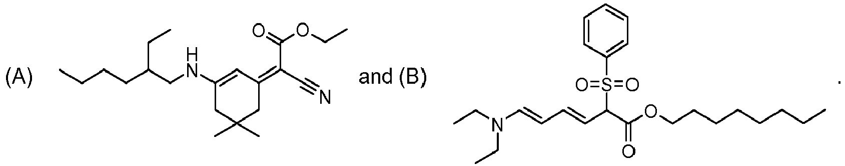 (A) and (B) can be either in E- or Z-configuration.

Each of the above-mentioned light-protective agents, especially the light-protective agents in the above Tables indicated as being preferred, can be used in admixture with the UV absorber combination according to the invention. It will be understood in that connection that, in addition to the UV absorber combination according to the invention, it is also possible for more than one of the additional light-protective agents to be used, for example, two, three, four, five or six further light-protective agents. Preference is given to the use of mixing ratios of UV absorbers according to the invention/further light-protective agents of from 1:99 to 99:1, especially from 1:95 to 95:1 and preferably from 10:90 to 90:10, based on weight. Of special interest are mixing ratios of from 20:80 to 80:20, especially from 40:60 to 60:40 and preferably of approximately 50:50. Such mixtures can be used, *inter alia,* to improve solubility.

Appropriate mixtures can be used especially advantageously in a cosmetic composition according to the invention.

The cosmetic compositions contain, for example, from 0.1 to 30 % by weight, preferably from 0.1 to 15 % by weight and especially from 0.5 to 10 % by weight, based on the total weight of the composition, of the UV absorber combination according to the present invention and at least one cosmetically tolerable adjuvant.

The cosmetic compositions can be prepared by physically mixing the UV absorbers with the adjuvant using customary methods, for example by simply stirring together the individual components, especially by making use of the dissolution properties of already known cosmetic UV absorbers, for example OMC, salicylic acid isooctyl ester, *inter alia.* The UV absorber can be used, for example, without further treatment.

The compositions according to the invention may in addition contain, as further adjuvants and additives, mild surfactants, super-fatting agents, pearlescent waxes, consistency regulators, thickeners, polymers, silicone compounds, fats, waxes, stabilisers, biogenic active ingredients, deodorising active ingredients, anti-dandruff agents, film formers, swelling agents, antioxidants, hydrotropic agents, preservatives, insect repellents, self-tanning agents, solubilizers, perfume oils, colorants, bacteria-inhibiting agents and the like.

Cosmetic formulations according to the invention are contained in a wide variety of cosmetic preparations. There come into consideration, for example, especially the following preparations: skin-care preparations, skin-tanning preparations, depigmenting preparations or insect-repellents
The cosmetic preparation according to the invention is distinguished by excellent protection of human skin against the damaging effect of sunlight.

The present invention will now be illustrated with the aid of examples, which should not however, under any circumstances, be interpreted as limiting as regards the scope of the invention.

In the text herein below, except where otherwise specified, the percentages
indicated are percentages by weight.

### Examples

### Example 1: Sun Protection Factors of Oleogels - Influence of the oil polarity of a sunscreen formulation on its sun protection factor

Oleogels of constant UV absorber composition but differing in emollient polarity are prepared. In a first trial, six formulations with different oils are sent to in vivo sun protection factor (SPF) determination. They all show extremely high SPF-values combined with a very high variability, making a meaningful evaluation impossible.

Using the same UV absorber composition, two oils are selected and the gel-former concentration is varied from zero up to the concentration used in the first study and one intermediate concentration in addition, resulting in six different samples. Again, the formulations are sent for in vivo SPF determination.

As oils Dibutyl Adipate and Diethylhexyl Carbonate (stationary phase) are used, the properties of which are listed in Table EX1. The results are summarized in Table EX2 and the complete information for the six formulations is shown in Table EX3.

| Table EX1: Oil properties | | | |
|---|---|---|---|
| | Interf. tension to water, γ (mN/m) | Log P_{octanol/water} | δ□(MPa^{1/2}) |
| Dibutyl Adipate | 14.3 | 3.9 | 18.9 |
| Diethylhexyl Carbonate | 29.1 | 6.8 | 17.1 |

The smaller the interfacial tension of the oil towards water [1], the more polar it is. This is in line with the values of Log P_{octanol/water}. The calculated Hildebrand solubility parameters δ are quite similar for both oils, indicating that their solubilizing capacities should be comparable.

As gel-former Steralkonium Hectorite (Bentone 27) is used in combination with Propylene Carbonate. Bentone 27 is a hydrophobically modified sheet silicate. The average dimensions of a clay platelet are 80 x 800 x 1 nm. Addition of Propylene Carbonate helps in developing the network structure of the gel-forming agent in the oil.

### Results of in vivo SPF Measurements

The UV absorber composition employed in all formulations is 5% Uvinul A plus, 2.5% Uvinul T150 and 3% Tinosorb S. Using the latest version of the BASF Sunscreen Simulator, for this composition a calculated SPF of 20.8 is obtained.

The results of the in vivo SPF screenings, performed in two testing institutions, are shown in Table 2.

| Table EX2: Results of in vivo SPF Screenings | | | |
|---|---|---|---|
| Concentration of Bentone 27 | 0 % | 6.5 % | 13 % |
| SPF with Dibutyl Adipate | 7.1 ± 2.9 | 27.7 ± 7.3 | 30.8 ± 7.6 |
| SPF with Diethylhexyl Carbonate | 10.8 ± 1.4 | 28.4 ± 5.7 | 24.1 ± 6.9 |

It is evident from Table EX2 that the formulations without the gel-forming agent show SPF values much smaller than expected from the calculation, whereas addition of gel-former leads to a dramatic increase of the SPF, exceeding the expected value. Obviously, it does not matter for the in vivo results, whether the concentration of Bentone 27 is 6.5% or 13%.

| Table EX3: Compositions of Formulations and in vivo SPF Screening Results | | | | | | | | |
|---|---|---|---|---|---|---|---|---|
| | INCI-Name | | OG-01 | OG-02 | OG-03 | OG-04 | OG-05 | OG-06 |
| | | | (%) | (%) | (%) | (%) | (%) | (%) |
| Part A | Diethylamino Hydroxybenzoyl Hexyl Benzoate | | 5,00 | 5,00 | 5,00 | 5,00 | 5,00 | 5,00 |
| | Ethylhexyl Triazone | | 2,50 | 2,50 | 2,50 | 2,50 | 2,50 | 2,50 |
| | Bis-Ethylhexyloxyphenol Methoxyphenyl Triazine | | 3,00 | 3,00 | 3,00 | 3,00 | 3,00 | 3,00 |
| | Dibutyl Adipate | | 86,80 | 78,15 | 69,50 | | | |
| | Diethylhexyl Carbonate | | | | | 86,80 | 78,15 | 69,50 |
| Part B | Stearalkonium Hectorite | | | 6,50 | 13,00 | | 6,50 | 13,00 |
| Part C | Propylene Carbonate | | | 2,15 | 4,30 | | 2,15 | 4,30 |
| In vivo SPF Screening Results | | Proderm | 7,1 ± 2,9 | 27,7 ± 7,3 | 30,8 ± 7,6 | 10,8 ± 1,4 | 28,4 ± 5,7 | 24,1 ± 6,9 |
| | | Institute Schrader | 4,9 ± 2,2 | n.d. | 56,7 ± 15,9 | 8,3 ± 1,9 | n.d. | 30,4 ± 6,8 |

### Results of Viscosity Measurements

For low viscosities a Brookfield DV-III with LV spindles was used, and for high viscosities a Brookfield DV-III Ultra with RV spindles.

| Table EX4: Results of Viscosity Measurements | | | | |
|---|---|---|---|---|
| Concentration of Bentone 27 | 0 % | 3.25 % | 6.5 % | 13 % |
| Viscosity with Dibutyl Adipate | 18 mPa·s | 32 mPa·s | 513 mPa·s | 5.10⁵ mPa·s |
| Viscosity with Diethylhexyl Carbonate | 21 mPa·s | 62 mPa·s | 1560 mPa·s | n.d. |

The results in Table EX4 show a dramatic increase of the viscosity with higher gel-former concentrations.

## Claims

1. Use of oleogels (a) for increasing the sun protection factor of sunscreens comprising at lease one organic or inorganic UV filter (b)

2. Use according to claim 1 wherein the stationary phase of the oleogels (a) are selected from
(sp₁) Guerbet alcohols,
(sp₂) esters of linear C₆-C₂₄ fatty acids with linear C₃-C₂₄ alcohols,
(sp₃) esters of branched C₆-C₁₃carboxylic acids with linear C₆-C₂₄ fatty alcohols,
(sp₄) esters of linear C₆-C₂₄ fatty acids with branched alcohols,
(sp₅) esters of hydroxycarboxylic acids with linear or branched C₆-C₂₂ fatty alcohols,
(sp₆) esters of linear and/or branched fatty acids with polyhydric alcohols,
(sp₇) triglycerides based on C₆-C₁₀ fatty acids,
(sp₈) liquid mono-/di-/tri-glyceride mixtures based on C₆-C₁₈ fatty acids,
(sp₉) esters of C₆-C₂₄ fatty alcohols and/or Guerbet alcohols with aromatic carboxylic acids,
(sp₁₀) esters of C₂-C₁₂dicarboxylic acids with linear or branched alcohols having from 1 to 22 carbon atoms or polyols having from 2 to 10 carbon atoms and from 2 to 6 hydroxy groups,
(sp₁₁) vegetable oils,
(sp₁₂) branched primary alcohols,
(sp₁₃) substituted cyclohexanes,
(sp₁₄) linear and branched C₆-C₂₂ fatty alcohol carbonates,
(sp₁₅) Guerbet carbonates,
(sp₁₆) esters of benzoic acid with linear and/or branched C₆-C₂₂alcohols,
(sp₁₇) linear or branched, symmetric or asymmetric dialkyl ethers having a total of from 12 to 36 carbon atoms,
(sp ₁₈) silicone oils,
(sp₁₉) aliphatic or naphthenic hydrocarbons,
(sp₂₀) monoesters of fatty acids with alcohols having from 3 to 24 carbon atoms,
(sp₂₁) isopropyl myristate,
(sp₂₂) isononanoic acid C₁₆-C₁₈alkyl esters,
(sp₂₃) stearic acid 2-ethylhexyl ester,
(sp₂₄) cetyl oleate,
(sp₂₅) glycerol tricaprylate,
(sp₂₆) coconut fatty alcohol caprinate/caprylate
(sp₂₇) n-butyl stearate
(sp₂₈) dicarboxylic acid esters,
(sp₂₉) diol esters,
(sp₃₀) polyols and
(sp₃₁) di- and/or trivalent metal salts.

3. Use according to claim 1 or 2, wherein the stationary phase of the oleogels (a) is selected from Dibutyl Adipate and Diethylhexyl Carbonate.

4. Use according to any of claims 1 to 3, wherein the gel former of the oleogels (a) is selected from
(gf₁) stearalkonium hectorite (bentonite),
(gf₂) gelatine,
(gf₃) silica,
(gf₄) montmorillonite,
(gf₅) monoglyceridees and diglycerides,
(gf₆) polysaccharides,
(gf₇) pectins and
(gf₈) specific polymers.

5. Use according to any of claims 1 to 4, wherein the gel former of the oleogels (a) is selected from (gf₁) stearalkonium hectorite in combination with propylene carbonate.

6. Use according to any of claims 1 to 5, wherein the oleogel (a) is formed from the stationary phase components (sp₂₉) dicarboxylic acid esters or (sp₁₄) linear and branched C₆-C₂₂ fatty alcohol carbonates and the gel former (gf₁) stearalkonium hectorite and (gf₈) specific polymers, preferably polyethylene.

7. Use according to any of claims 1 to 6, wherein the UV filters (b) are selected from
(b₁) triazine derivatives,
(b₂) hydroxybenzophenone derivatives,
(b₃) Methoxydibenzoylmethane derivatives,
(b₄) substituted acrylates,
(b₅) cinnamic acid derivatives,
(b₆) salicylic acid derivatives,
(b₇) benzotriazole derivatives; and
(b₈) inorganic pigments.

8. Use according to any of claims 1 to 7, wherein the triazine derivatives (b₁) are selected from compounds of formula 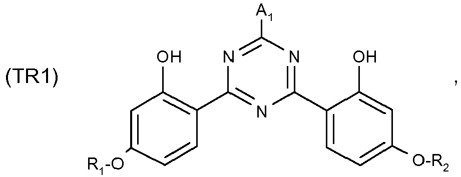 wherein
R₁ and R₂ are each independently of the other C₁-C₁₈alkyl; C₂-C₁₈alkenyl; or a radical of formula -CH₂-CH(-OH)-CH₂-O-T1;
A₁ is a radical of formula 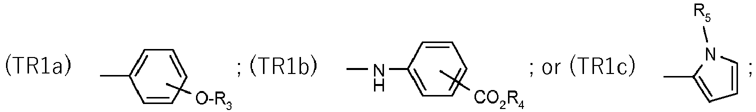
R₃ is hydrogen; or C₁-C₁₀alkyl,
R₄ is hydrogen; M; or C₁-C₅alkyl;
R₅ is C₁-C₁₈alkyl; and
M is a metal cation.

9. Use according to any of claims 1 to 8, wherein the triazine derivatives (b₁) correspond the compounds of formula

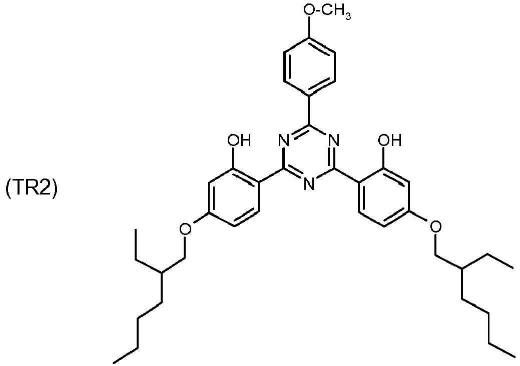

10. Use according to any of claims 1 to 7 wherein the triazine derivatives (b₁) correspond to the compounds of formula 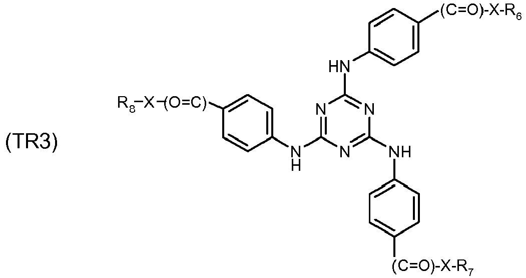 wherein
R₆, R₇ and R₈ independently of each other are C₁-C₂₀alkyl, C₆-C₁₀aryl, heteroaryl, optionally substituted;
X is O; or NR₉; and
R₉ is hydrogen; C₁-C₂₀alkyl, C₆-C₁₀aryl, heteroaryl, optionally substituted,

11. Use according to claim 1 to 7 or 10, wherein the triazine derivatives (b₁) correspond to the compounds of formula

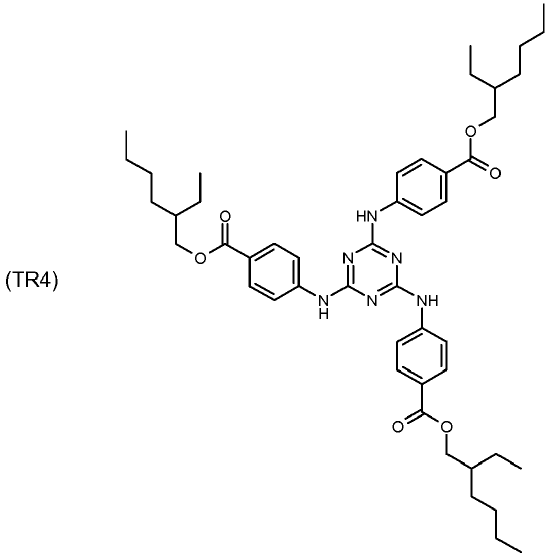

12. Use according to any of claims 1 to 7 or 10, wherein the triazine derivatives (b₁) correspond to the compounds of formula

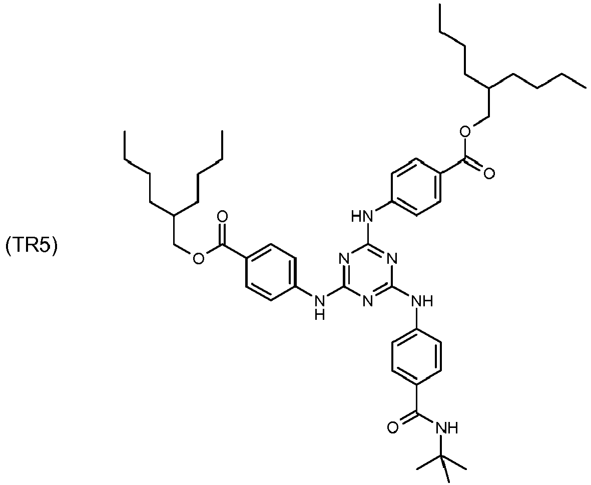

13. Use according to any of claims 1 to 7, wherein the hydroxybenzophenone derivatives (b₂) correspond to the formula 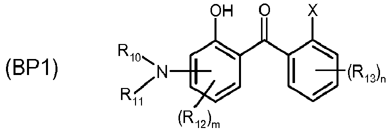 wherein
R₁₀ and R₁₁, independently from each other are hydrogen, C₁-C₂₀alkyl, C₂-C₁₀alkenyl, C₃-C₁₀cycloalkyl, C₃-C₁₀cycloalkenyl, where the substituents R₁₀ and R₁₁ together with the nitrogen atom to which they are bonded may form a 5- or 6-membered ring;
R₁₂ and R₁₃ independently from each other are C₁C₂₀alkyl; C₂-C₁₀alkenyl; C₃-C₁₀cycloalkyl; C₃-C₁₀cycloalkenyl; C₁-C₁₂alkoxy; C₁-C₂₀alkoxycarbonyl; C₁-C₁₂alkylamino; C₁-C₁₂dialkylamino; aryl; heteroaryl, optionally substituted; substituents which confer solubility in water, chosen from the group consisting of a nitrile group, carboxylate, sulfonate and ammonium radicals;
X is hydrogen; COOR₁₄; or CONR₁₅R₁₆;
R₁₄ to R₁₆ are hydrogen; C₁-C₂₀alkyl; C₂-C₁₀alkenyl; C₃-C₁₀cycloalkyl; C₃-C₁₀cycloalkenyl, or -(Y-O)o-Z, C₆-C₁₀aryl;
Y is -(CH₂)₂-; -(CH₂)₃-, -(CH₂)₄-; or -CH(CH₃)-CH₂-;
Z is -CH₂-CH₃; -CH₂-CH₂-CH₃; -CH₂-CH₂-CH₂-CH₃; or -CH(CH₃)-CH₃;
m is from 0 to 3;
n is from 0 to 4; and
o is from 1 to 20.

14. Use according to claim 1 to 7 or 13, wherein the hydroxybenzophenone derivatives (b) correspond to the formula

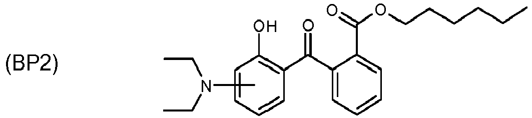

15. Use according to any of claims 1 to 7, wherein the methoxydibenzoylmethane derivatives (b₃) correspond to the formula

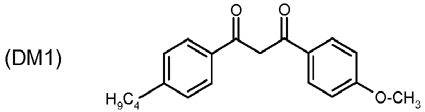

16. Use according to any of claims 1 to 7, wherein the substituted acrylates (b₄) correspond to the formula

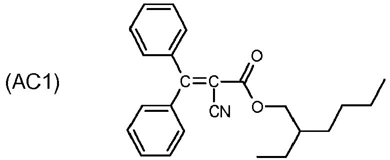

17. Use according to any of claim 1 to 7, wherein the cinnamic acid derivatives (b₅) correspond to the formula

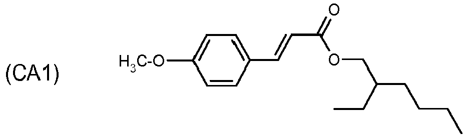

18. Use according to any of claim 1 to 7, wherein the salicylic acid derivatives (b₆) correspond to the formula

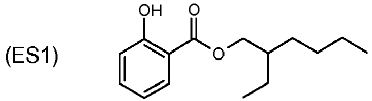

19. Use according to any of claim 1 to 7, wherein the benzotriazole derivatives (b₇) corresponds to formula

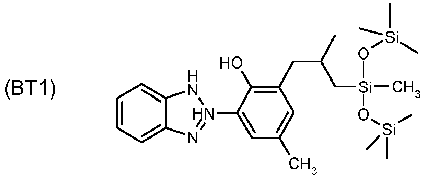

20. Use according to any of claims 1 to 7, wherein the inorganic pigments (b₈) are selected from oil-dispersed TiO₂.

21. Use according to any of claims 1 to 20 wherein the UV filters (b) are used in mixtures.

22. Use according to any of claims 1 to 21, wherein mixtures of (Mix01) compound (TR2) and compound (CA1); (Mix02) compound (TR2) and compound (TR4); (Mix03) compound (TR2) and compound (TR5); (Mix04) compound (TR2) and compound (AC1); (Mix05) compound (BP1) and compound (CA1); (Mix06) compound (BP1) and compound (TR4); (Mix07) compound (BP1) and compound (TR5); (Mix08) compound (BP1) and compound (AC1); (Mix09) compound (TR2) and compound (BP1); (Mix10) compound (TR4) and compound (CA1); (Mix11) compound (TR5) and compound (CA1); (Mix12) compound (DM1) and compound (TR4); (Mix13) compound (DM1) and compound (TR5); (Mix14) compound (DM1) and compound (AC1); (Mix15) oil-dispersed TiO₂ and compound (TR2); (Mix16) oil-dispersed TiO₂ and compound (BP1); (Mix17) oil-dispersed TiO₂ and compound (TR4); (Mix18) oil-dispersed TiO₂ and compound (TR5); (Mix19) oil-dispersed TiO₂ and compound (CA1); (Mix20) oil-dispersed TiO₂ and compound (AC1); (Mix21) oil-dispersed ZnO and compound (TR2); (Mix22) oil-dispersed ZnO and compound (TR2); (Mix23) oil-dispersed ZnO and compound (BP1); (Mix24) oil-dispersed ZnO and compound (TR4); (Mix25) oil-dispersed ZnO and compound (TR5); (Mix26) oil-dispersed TiO₂ and oil-dispersed ZnO; (Mix27) compound (DM1) and compound (AC1); (Mix 28) compound (DM1) and compound (TR2); (Mix29) compound (DM1) and compound (AC1); and compound (TR2); (Mix30) compound (DM1) and compound (AC1); and compound (TR4); (Mix31) compound (DM1) and compound (AC1); and compound (TR5); (Mix32) compound (CA1) and compound (BP2); and compound (TR4); (Mix33) compound (CA1) and compound (BP2); and compound (TR5); (Mix34) compound (CA1) and compound (BP2); and compound (TR2); (Mix35) compound (DM1) and compound (BP3); and compound (AC1); (Mix36) compound (CA1) and compound (BP2); and compound (BP3); (Mix37) compound (TR2) and compound (TR4); and compound (BP2); (Mix38) compound (DM1) and compound (AC1); and compound (TR2); and compound (TR4); (Mix39) compound (DM1) and compound (AC1); and compound (TR2); and compound (TR5); (Mix40) compound (CA1) and compound (BP2); and compound (TR2); and compound (TR4); (Mix41) compound (CA1) and compound (BP2); and compound (TR2); and compound (TR5); (Mix42) compound (CA1) and compound (BP2); and compound (BP3); and compound (TR2); (Mix43) compound (CA1) and compound (BP2); and compound (TR2); and compound (TR5); (Mix44) compound (CA1) and compound (BP2); and compound (TR2); and compound (TR4); and compound (ES1); (Mix45) compound (CA1) and compound (BP2); and compound (TR2); and compound (TR5); and compound (ES1); (Mix46) compound (DM1) and compound (AC1); and compound (TR2); and compound (TR4); and compound (ES1); (Mix47) compound (CA1) and compound (AC1); and compound (TR2); and compound (TR5); and compound (ES1);
are used.

23. Use according to any of claims 1 to 22 wherein mixtures of (Mix 37) comprising the compound (TR2) and compound (TR4) and compound (BP2) are used.

24. Use according to any of claims 1 to 23 wherein
(a) the oleogel formed from (gf₁) stearalkonium hectorite and the stationry phase selected from Dibutyl Adipate and Diethylhexyl Carbonate; and
(b) (Mix 37) comprising the compound (TR2) and compound (TR4) and compound (BP2) is used.

25. Cosmetic composition, comprising
(a) a stable oleogel and
(b) at least one organic or inorganic UV filter.
